# EUROPÄISCHE PATENTSCHRIFT

(11) **EP 1 171 424 B1**
(45) Veröffentlichungstag und Bekanntmachung des Hinweises auf die Patenterteilung: **27.05.2009**
(21) Anmeldenummer: 00918867.3
(22) Anmeldetag: 11.04.2000
(51) Int. Cl.: C07D 235/06, C07D 403/08, C07D 401/08, A61K 31/4184, A61P 25/00

(54) **CYCLOALKYLSUBSTITUIERTE BENZIMIDAZOLE UND IHRE VERWENDUNG ALS PARP INHIBITOREN**
CYCLO-ALKYL SUBSTITUTED BENZIMIDAZOLES AND THEIR USE AS PARP INHIBITORS
BENZIMIDAZOLES A SUBSTITUTION CYCLOALKYLE, ET LEUR UTILISATION COMME INHIBITEURS DE LA PARP

(30) Priorität: 22.04.1999 DE 19918211
(43) Veröffentlichungstag der Anmeldung: 16.01.2002
(73) Patentinhaber: Abbott GmbH & Co. KG, 65205 Wiesbaden (DE)
(72) Erfinder: LUBISCH, Wilfried, D-69115 Heidelberg (DE); KOCK, Michael, D-67105 Schifferstadt (DE); HÖGER, Thomas, D-68535 Edingen-Neckarhausen (DE); GRANDEL, Roland, D-69221 Dossenheim (DE); SCHULT, Sabine, D-67346 Speyer (DE); MÜLLER, Reinhold, D-67105 Schifferstadt (DE)
(74) Vertreter: Grünecker, Kinkeldey, Stockmair & Schwanhäusser Anwaltssozietät
(86) Internationale Anmeldenummer: PCT/EP2000/003231
(87) Internationale Veröffentlichungsnummer: WO 2000/064878

(56) Entgegenhaltungen:
- WO-A-97/04771
- WO-A-98/33802

## Beschreibung

Die vorliegende Erfindung betrifft neuartige Benzimidazole, ihre Herstellung und die Verwendung als Inhibitoren des Enzyms Poly(ADP-ribose)polymerase oder PARP (EC 2.4.2.30) zur Herstellung von Arzneimitteln.

Poly(ADP-ribose)polymerase (PARP) bzw. wie es auch genannt wird Poly(ADP-ribose)synthase (PARS) stellt ein regulatorisches Enzym dar, das in Zellkernen gefunden wird (K. Ikai et al., J. Histochem. Cytochem. 1983, 31, 1261-1264). Man nimmt an, daß PARP eine Rolle bei der Reparatur von DNA-Brüchen spielt (M.S. Satoh et al., Nature 1992, 356, 356-358). Schädigungen oder Brüche der DNA-Stränge aktivieren das Enzym PARP, das, wenn es aktiviert ist, die Übertragung von ADP-Ribose aus NAD katalysiert (S. Shaw, Adv.Radiat.Biol., 1984, 11, 1-69). Dabei wird Nikotinamid aus NAD freigesetzt. Nikotinamid wird unter Verbrauch des Energieträgers ATP von anderen Enzymen wieder in NAD umgewandelt. Eine Überaktivierung von PARP hätte dementsprechend einen unphysiologisch hohen Verbrauch von ATP zur Folge und dies führt im Extremfall zu Zellschädigungen und Zelltod.

Es ist bekannt, daß Radikale wie Superoxid-Anion, NO und Wasserstoffperoxid in Zellen zu DNA-Schädigungen führen können und damit PARP aktivieren. Die Bildung von großen Mengen an Radikalen wird bei eine Reihe von pathophysiologischen Zuständen beobachtet und man geht davon aus, daß diese Anhäufung von Radikalen zu den beobachteten Zell- bzw Organschäden führen oder beitragen. Dazu zählt von zum Beispiel ischämische Zustände von Organen wie im Schlaganfall, Herzinfarkt (C. Thiemermann et al., Proc.Natl. Acad.Sci.USA, 1997, 94, 679-683) oder Ischämie der Nieren, aber auch Reperfusionsschäden wie sie zum Beispiel nach der Lyse von Herzinfarkt auftreten (s. oben : C. Thiemermann et al.). Die Hemmung von dem Enzym PARP könnte demzufolge ein Mittel sein, um diese Schäden zum mindestens zum Teil zu verhindern oder abzumildern. PARP-Inhibitoren könnten somit ein neues Therapieprinzip zur Behandlung von eine Reihe von Krankheiten darstellen.

Das Enzym PARP beeinflußt die Reparatur von DNA-Schäden und könnte somit auch in der Therapie von Krebs-Erkrankungen eine Rolle spielen, da in Kombination mit cytostatisch wirksamen Stoffen ein höheres Wirkpotential gegenüber Tumorgewebe beobachtet wurde (G. Chen et al. Cancer Chemo.Pharmacol. 1988, 22, 303).

Nicht limitierende Beispiele für Tumoren sind Leukämie, Gliokastome, Lymphome, Melanome, Mama- und Cervialkakrzinome.

Zudem wurde gefunden, daß PARP-Inhibitoren immunosuppressive Wirkung zeigen können (D. Weltin et al. Int.J.Immunopharmacol. 1995, 17, 265-271).

Es wurde ebenfalls entdeckt, daß PARP bei immunologischen Erkrankungen bzw. Krankheiten, in denen das Immunsystem eine wichtige Rolle spielt, wie zum Beispiel rheumatoide Arthritis und septischer Schock, involviert ist, und daß PARP-Inhibitoren einen günstigen Effekt auf den Krankheitsverlauf zeigen können (H. Kröger et al. Infammation 1996, 20, 203-215; W. Ehrlich et al. Rheumatol. Int. 1995, 15, 171-172; C.Szabo et al., Proc.Natl.Acad.Sci.USA 1998, 95, 3867-3872; S. Cuzzocrea et al. Eur.J.Pharmacol. 1998, 342, 67-76).

Unter PARP im Sinne dieser Erfindung werden auch Isoenzyme des oben beschriebenen PARP-Enzyms verstanden.

Weiterhin zeigte der PARP-Inhibitor 3-Aminobenzamid protektive Effekte in einem Model für den Kreislaufschock (S. Cuzzocrea et al., Br.J.Pharmacol. 1997, 121, 1065-1074).

Ebenfalls gibt es experimentelle Hinweise, das Inhibitoren des Enzymes PARP als Mittel zur Behandlung von Diabetes mellitus nützlich sein könnten (V. Burkart et al. Nature Med. 1999, 5, 314-319).

Benzimidazole sind vielfach beschrieben worden. So sind in DE 38 30 060 alkylierte Derivate als Inhibitoren der Erythrozytenaggregation offengelegt. In DE 35 22 230 ist ein Ester-Derivat vom 2-Phenylbenzimidazol als Inhibitor der Plättchenaggragation aufgeführt. Halogen-substituierte 2-Phenylbenzimizazole, die am Phenyl-Ring substituierte Amin-Reste tragen, sind in WO 98/06703 als MCP-1-Antagonisten beschrieben worden.

Ebenfalls sind 2-Phenyl-benzimidazole bekannt, bei denen die Benzimidazol-Gruppe durch eine Amid-Gruppe substituiert ist. 5-Amido-Derivate des 2-Phenylbenzimidazols, die am Phenyl-Ring Alkyloxy-Reste tragen, sind in WO 94/12461 als Inhibitoren der cAMP-Phosphodiesterase beschrieben worden. Für analoge Derivate wurde in DE 35 46 575 (z.B. Beispiel 15) gefunden, daß diese Verbindungen positiv inotrope Effekte auslösen. Ebenfalls 4-Amido-Derivate, die in 3-Stellung ein Pyridyl-Rest tragen, sind in WO 97/48697 als Inhibitoren der cAMP-Phosphodiesterase aufgeführt.

Die Synthese von 2-Phenyl-benzimidazyl-4-amiden ist in J.Chem.Soc. Perkin Trans 1, 1979, 2303-2307 beschrieben worden. Analoge Verbindungen, die am Amid-Rest noch eine substituierte Alkyl-Kette tragen, und die cytotoxische Wirkung haben sollen, sind in J.Med.Chem. 1990, 33, 814-819 aufgeführt. In WO 97/04771 sind dagegen Benzimidazol-4-amide aufgeführt, die das PARS hemmen. Insbesondere sind Derivate dort als wirksam beschrieben, die einen Phenyl-Ring in 2-Stellung tragen, wobei der Phenyl-Ring noch mit einfachen Substituenten wie Nitro, Methoxy und CF₃, substituiert sein kann. Obwohl diese Substanzen zum Teil gute Hemmung des Enzyms PARP zeigen, haben die dort beschrieben Derivate als Nachteil, daß sie nur wenig oder keine Löslichkeit in wäßrigen Lösungen zeigen und somit nicht als wäßrige Lösung appliziert werden können.

Benzimidazole, die in 2-Stellung Cycloalkyl-Reste tragen, sind ebenfalls schon beschrieben worden. So sind in F. Pellicciari et al., Arch. Pharm. 1985, 318, 393-399 2-Cyclohexyl-Derivate erwähnt, die in 1-Stellung noch Alkylamide tragen können oder in Ann. 1952, 575, 162, wo auch Methyl-Derivate dargestellt wurden, bei denen die Methyl-Gruppe am Benzimidazol-Aromaten stehen. 2-Cycloalkyl-Benzimidazole, bei denen der aromatische Ring mit Chlor- oder Nitro-Gruppen substituiert ist sind zum Beispiel in DE 2649125, E. Seuer et al., Farmaco 1997, 52, 99 und M. Benchidmi et al, Bull. Soc. Chim. Belg. 1995, 104, 605-612 beschrieben. Derivate der Benzimidazol-5-carbonsäure, in 2-Stellung Cyclopentandion-Reste tragen, sind in Ann., 1893, 273, 320 erwähnt. Benzimidazole, wobei am aromatischen Ring Laktam-Ringe anelliert sind, wurden in DE 2732951 und in W. Saal et al., J.Med.Chem. 1989, 32, 1481-1491 dargestellt. Es wurden jedoch noch nie Benzimidazole mit Carbocyclischen-Ringen in 2-Stellung beschrieben, die am Benzimidazol-Ring oder insbesondere in 4-Stellung am Benzimidazol-Ring eine Amid-Gruppe trägt.

In einer Reihe von Therapien wie Schlaganfall werden die Wirkstoffe intravenös als Infusionslösung appliziert. Dazu ist es notwendig Substanzen, hier PARP-Inhibitoren, zur Verfügung zu haben, die ausreichende Wasserlöslichkeit bei physiologischen pH-Werten oder angenäherten pH-Werten (z.B: pH-Werten von 5-8) aufweisen, so daß eine Infusionslösung hergestellt werden kann. Viele der beschriebenen PARP-Inhibitoren, insbesondere die besser wirksamen PARP-Inhibitoren, haben jedoch den Nachteil, daß sie nur geringe oder keine Wasserlöslichkeit bei diesen pH-Werten zeigen und somit nicht für eine intravenöse Applikation in Frage kommen. Derartige wirkstoffe können nur mit Hilfsstoffen, die die wasserlöslichkeit vermitteln sollen, appliziert werden (vgl. WO 97/04771). Diese Hilfsstoffe, zum Beispiel Polyethylenglykol und Dimethysulfoxid, verursachen häufig Nebeneffekte oder sind sogar unverträglich. Gut wirksame PARP-Inhibitoren mit ausreichender Wasserlöslichkeit sind bisher nicht beschrieben worden.

Die WO 98/33802 beschreibt Chinazolinonverbindungen, die in der Lage sind, die Aktivität von poly ADP-ribosyltransferase (PARP) zu hemmen.

Es wurde überraschenderweise gefunden, daß Benzimidazole, die am Imidazol-Ring einen gesättigten oder einfach ungesättigten Carbocyclus tragen, gut wirksame Inhibitoren darstellen, die aber durch die weiteren Einbau von aliphatischen Amin-Resten eine Salzbildung mit Säuren ermöglichen und dadurch eine deutlich verbesserte Wasserlöslichkeit zeigen.

In der vorliegenden Erfindung werden neue Benzimidazole-Derivate der allgemeinen Formel I bzw. II beschrieben, die potente PARP-Inhibitoren darstellen und zum Teil auch ausreichende Wasserlöslichkeit zeigen, die eine Applikation als Infusionslösung ermöglicht.

Gegenstand der vorliegenden Erfindung sind substituierte Benzimidazole der allgemeinen Formeln I und II: 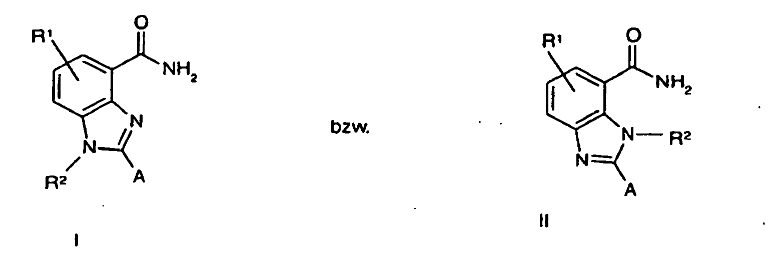 worin
- A: einen gesättigten oder einfach ungesättigten Carbozyklus mit 3 bis 8 Kohlenstoffatomen bedeutet, der zusätzlich noch einen Benzolring ankondensiert haben kann, wobei die Ringe noch mit ein oder zwei unterschiedlichen oder gleichen Resten R³ sowie dem Rest R⁴ substituiert sein können, und
- R¹: Wasserstoff, und
- R²: Wasserstoff, verzweigtes und unverzweigtes C₁-C₆-Alkyl, C₁-C₄-Alkyl-Phenyl und
- R³: C₁-C₆-Alkyl, OH, O-C₁-C₄-Alkyl, O-C₁-C₄-Alkyl-Phenyl, NR¹¹R¹², Phenyl, C₁-C₄-Alkyl-Phenyl, CF₃, COOH, COOC₁-C₄-Alkyl, CONH-C₁-C₄-Alkyl, CONH₂, wobei die Phenyl-Ringe noch mit maximal zwei gleichen oder unterschiedlichen Resten R³¹ substituiert sein können, und wobei R¹¹ und R¹² unabhängig voneinander Wasserstoff oder C₁-C₄-Alkyl bedeuten und
- R³¹: OH, C₁-C₆-Alkyl, O-C₁-C₄-Alkyl, Chlor, Brom, Jod, Fluor, CF₃, Nitro, NR¹¹R¹², und
- R⁴: -(O)ₚ-(CH₂)_{q}-B bedeutet, wobei
- B: NR⁴¹R⁴² und 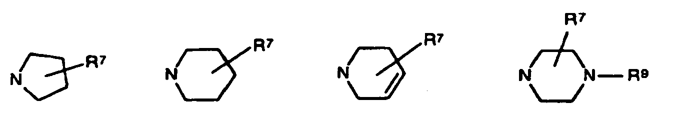 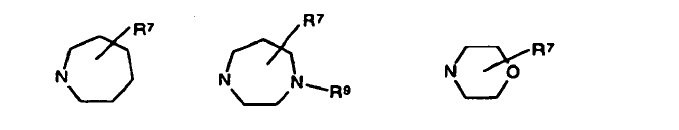 bedeutet, wobei
- p: 0 und 1 bedeuten kann und
- q: 0, 1, 2 oder 3 sein kann, wobei wenn q = 0 ist auch p = 0 ist, und
- R⁴¹: Wasserstoff, C₁-C₆-Alkyl, (CH₂)ᵣ-E und
- R⁴²: Wasserstoff, C₁-C₆-Alkyl, -CO-R⁸, SO₂-R⁸, -(C=N)-R⁸ und -(C=N)-NHR⁸ und
- r: 0, 1, 2, 3, 4 und
- E: Phenyl, der noch maximal zwei Reste R⁷² tragen kann, und, wenn r ≠ 0,1 ist, auch NR¹¹R¹², NH-C₁-C₄-Alkyl-Phenyl, Pyrrolidin, Piperidin, Dihydropiperidin, Morpholin, Homopiperidin, Piperazin, das noch mit C₁-C₆-Alkyl und C₁-C₄-Alkyl-Phenyl substituiert sein kann, und Homopiperazin, das noch mit C₁-C₆-Alkyl und C₁-C₄-Alkyl-Phenyl substituiert sein kann, und
- R⁷: Wasserstoff, C₁-C₆-Alkyl, Phenyl, wobei der Ring noch mit bis zu zwei gleichen oder unterschiedlichen Resten R⁷¹ substituiert sein kann, und
- R⁷¹: OH, C₁-C₆-Alkyl, O-C₁-C₄-Alkyl, Chlor, Brom, Jod, Fluor, CF₃, Nitro, NR¹¹R¹², und
- R⁷²: OH, C₁-C₆-Alkyl, O-C₁-C₄-Alkyl, Chlor, Brom, Jod, Fluor, CF₃, Nitro, NR¹¹R¹² , und
- R⁸: C₁-C₆-Alkyl, Phenyl, C₁-C₄-Alkyl-Phenyl-O-C₁-C₄-Alkyl-Phenyl, wobei der Ring noch mit bis zu zwei gleichen oder unterschiedlichen Resten R⁸¹ substituiert sein kann, und
- R⁸¹: OH, C₁-C₆-Alkyl, O-C₁-C₄-Alkyl, Chlor, Brom, Jod, Fluor, CF₃, Nitro, NR¹¹R¹², und
- R⁹: Wasserstoff, C₁-C₆-Alkyl, C₁-C₄-Alkyl-Phenyl, Phenyl, wobei die Ringe noch mit bis zu zwei Resten R⁹¹ substituiert sein kann, und
- R⁹¹: OH, C₁-C₆-Alkyl, O-C₁-C₄-Alkyl, Chlor, Brom, Jod, Fluor, CF₃, Nitro, NR¹¹R¹², und sein kann.

Bevorzugt sind bei A Carbozyklen, die mindestens einfach substituiert sind. Bevorzugte Carbozyklen sind: Tetralin, Indan, Cycloheptan, Cyclohexan, Cyclopentan, Cyclobutan und Cyclopropan.

Bevorzugt werden die Verbindungen der Formeln I und II, wobei A ein Cyclohexan-Ring darstellt , R¹, R² und R³ Wasserstoff darstellt und R⁴ die Bedeutung wie oben hat, wobei p 0 und 1 und q 0,1 und 2 sind, R⁴¹ und R⁴², unabhängig voneinander, Wasserstoff und C₁-C₄-Alkyl bedeutet, R⁷ Wasserstoff, C₁-C₄-Alkyl und Phenyl, R⁹ Wasserstoff, C₁-C₄-Alkyl und C₁-C₂-Alkyl-Phenyl, und R⁴ in 3-und 4-Stellung am Cyclohexan-Ring stehen kann, wobei sowohl die cis als auch die trans-Formen oder deren Gemische eingeschlossen sind.

Besonders bevorzugt sind die Verbindungen der allgemeinen Formeln I und II, wobei A für ein Cyclohexan.Ring steht und R¹, R² und R⁴ Wasserstoff ist und R⁴ die Bedeutung wie oben hat, wobei p 0 und 1 und q 0,1 und 2, R⁴¹ und R⁴², unabhängig voneinander, Wasserstoff und C₁-C₄-Alkyl bedeutet, R⁷ Wasserstoff, R⁹ Wasserstoff, C₁-C₄-Alkyl und Benzyl, und R⁴ in 4-Stellung am Cyclohexan-Ring stehen kann, wobei sowohl die cis- und die trans-Formen als auch deren Gemische eingeschlossen sind.

Die Verbindungen der Formel I und II können als Racemate, als enantiomerenreine Verbindungen oder als Diastereomere eingesetzt werden. Werden enantiomerereine Verbindungen gewünscht, kann man diese beispielsweise dadurch erhalten, daß man mit einer geeigneten optisch aktiven Base oder Säure eine klassische Racematspaltung mit den Verbindungen der Formel I und II oder ihren Zwischenprodukten durchführt.

Gegenstand der Erfindung sind auch zu Verbindungen der Formel I mesomere oder tautomere Verbindungen.

Ein weiterer Gegenstand der Erfindung sind die physiologisch verträglichen Salze der Verbindungen I und II, die sich durch Umsatz von Verbindungen I mit einer geeigneten Säure oder Base erhalten lassen. Geeignete Säuren und Basen sind zum Beispiel in Fortschritte der Arzneimittelforschung, 1966, Birkhäuser Verlag, Bd.10, S. 224-285, aufgelistet. Dazu zählen zum Beispiel Salzsäure, Citronensäure, Weinsäure, Milchsäure, Phosphorsäure, Methansulfonsäure, Essigsäure, Ameisensäure, Maleinsäure, Fumarsäure usw. bzw. Natriumhydroxid, Lithiumhydroxid, Kaliumhydroxid und Tris.

Unter Prodrugs werden solche Verbindungen verstanden, die in vivo in Verbindungen der allgemeinen Formel I und II metabolisiert werden. Typische Prodrugs sind Phosphate, Carbamate von Aminosäuren, Ester und andere.

Die Herstellung der erfindungsgemäßen Benzimidazole I und II kann auf verschiedenen Wegen erfolgen und wurde in den Syntheseschemata 1-3 skizziert.

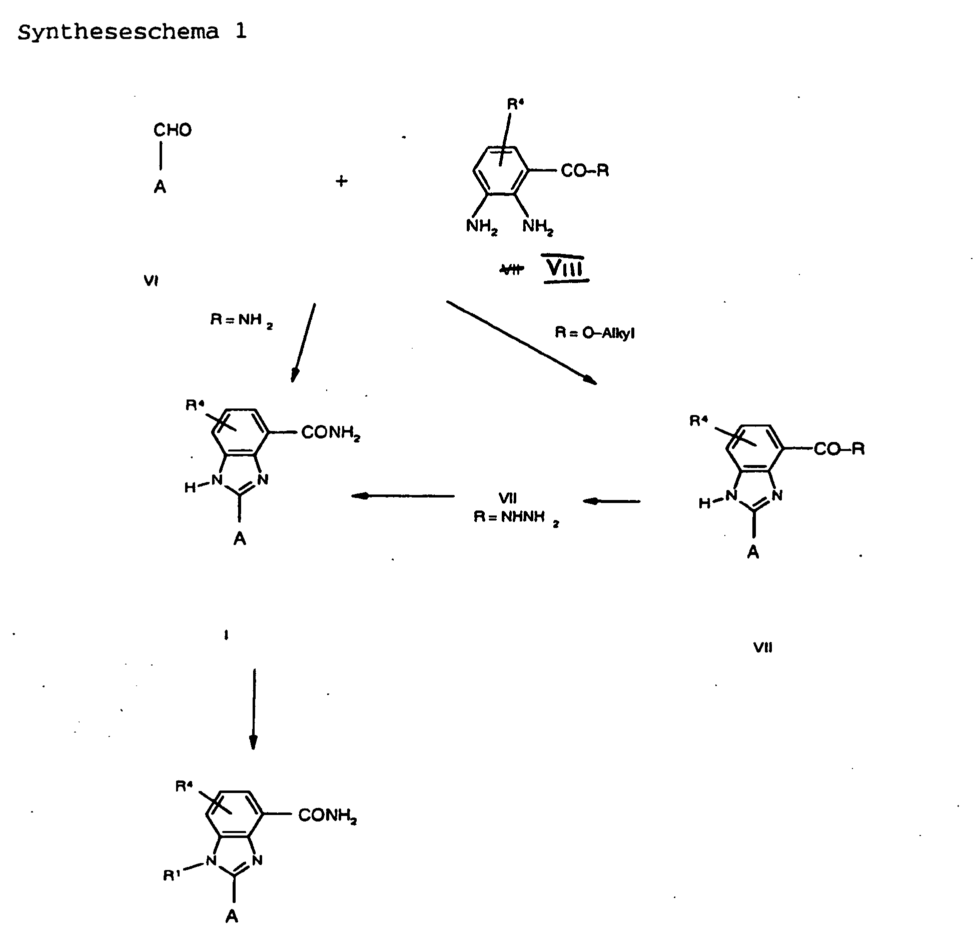

Durch Kondensation des Aldehyds mit Phenylendiaminen erhält man das Benzimidazol VII, wobei man bevorzugt in polaren Lösungsmitteln wie Ethanol oder Dimethylformamid und Zusatz von Säuren wie Essigsäure bei erhöhter Temperatur arbeitet, in der Regel 80-120°C. Günstig für die Reaktion ist der Zusatz von schwachen Oxidationsmittel wie Kupfer-II-Salzen, die als wäßrige Lösung zugesetzt werden.

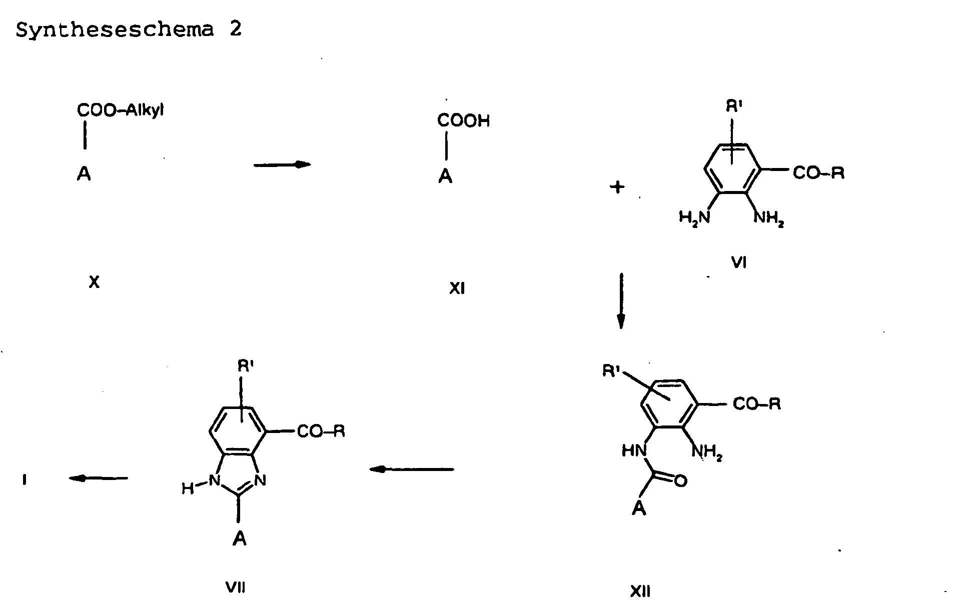

Wenn in dem Phenylendiamin VIII R = NH₂ ist, entstehen bei der Kondensation direkt erfindungsgemäße Verbindungen I. Ansonsten kann man, falls R = O-Alkyl ist, diesen Ester mit Ammoniak, bei gegebenenfalls erhöhter Temperatur und erhöhtem Druck, zum Amid I umsetzten. Alternativ kann man den Ester VII mit Hydrazin in polaren Lösungsmitteln wie die Alkohole Butanol und Ethanol oder auch Dimethylformamid, bei erhöhten Temperaturen, vorzugsweise 80-130°C. umsetzten, wobei ein Hydrazid VII (R = NHNH₂) anfällt, das danach noch unter reduktiven Bedingungen, wie mit Raney-Nickel in Alkoholen unter Rückfluß, zum Amid I reduziert werden kann.

Eine Einführung des Restes R2 am Benzimidazol-Rest in I (R2 =H) gelingt unter Alkylierungsbedingungen wie oben (siehe V-VI), wobei allerdings der Reaktionspartner R2-L (L = Abgangsgruppe wie oben) eingesetzt werden muß (siehe Schema 1).

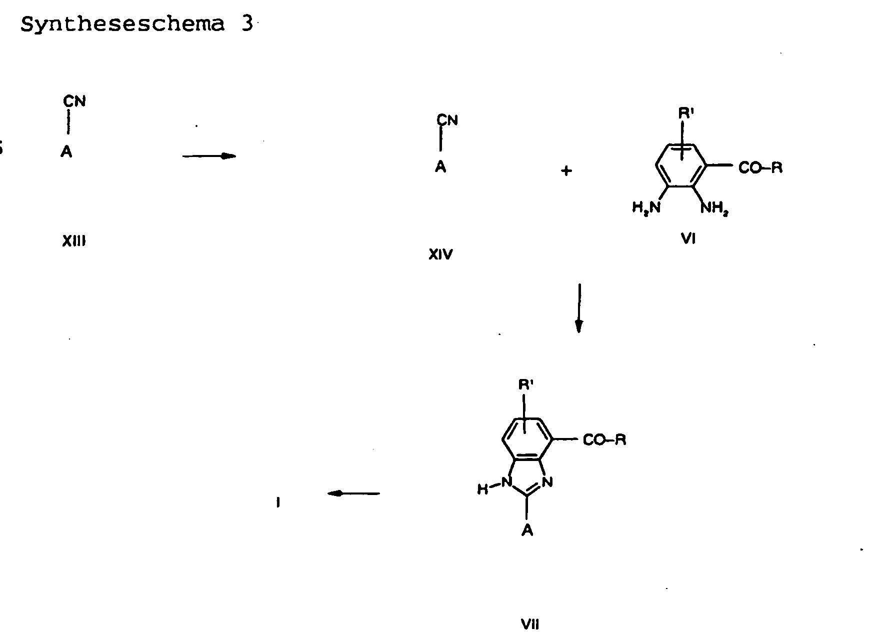

Alternativ zu den im Schema 1 gezeigten Aldehyden VI kann man auch Säuren wie XI (siehe Schema 2) oder Nitrile wie XIV(siehe Schema 3) anstelle des Benzaldehyds einsetzen. Die Herstellung dieser Derivate erfolgt analog zur Herstellung der substituierten Aldehyde VI. Ausgehend von XI erfolgt die Kondensation zu VII in zwei Stufen . Zuerst wird die Säure XI mit dem Anilin VI in einer peptidartigen Kupplung zum Amid XII umgesetzt. Dabei arbeitet man nach üblichen Bedingungen, die zum Beispiel im Houben-Weyl, Methoden der Organischen Chemie, 4. Aufl., E5, Kap. V bzw. C.R. Larock, Comprehensive Organic Transformations, VCH Publisher, 1989, Seite 972f. aufgelistet sind. Der Ringschluß erfolgt zum Benzimidazol erfolgt danach bei erhöhter Temperatur, zum Beispiel 60-180°C, mit oder ohne Lösungsmitteln wie Dimethylformamid, unter Zusatz von Säuren wie Essigsäure oder direkt in Essigsäure selbst.

Die Reaktion des Phenylendiamins VI mit einem Nitril XIV erfolgt ebenfalls unter üblichen Bedingungen. Dabei kann man in Lösungsmitteln wie Dimethylformamid unter Zusatz von Säuren oder auch in Polyphosphorsäure bei erhöhter Temperatur wie 60-200°C arbeiten. Allerdings kann man auch die üblichen Methoden zur Herstellung von Amidinen aus Nitrilen anwenden, wie sie in Houben-Weyl, Methoden der organischen Chemie, E5, S. 1304 f., J. Amer. Chem. Soc. 1957, 427 und J.Org.Chem. 1987, 1017 beschrieben sind.

Die oben genannten substituierten Benzimidazole I und II stellen Inhibitoren des Enzyms Poly(ADP-ribose)polymerase oder PARP (EC 2.4.2.30) dar.

Die inhibitorische Wirkung der vorher genannten substituierten Benzimidazole I und II kann mit einem in der Literatur bereits bekannten Enzymtest ermittelt werden, wobei als Wirkmaßstab ein Kᵢ-Wert ermittelt wird. Die Benzimidazole I und II wurden in dieser Weise auf Hemmwirkung des Enzyms Poly(ADP-ribose)polymerase oder PARP (EC 2.4.2.30) gemessen.

Die substituierten Benzimidazole der allgemeinen Formeln I und II stellen Inhibitoren der Poly(ADP-ribose)polymerase (PARP) bzw. wie es auch genannt wird Poly(ADP-ribose)synthase (PARS) dar und können somit zur Behandlung und Prophylaxe von Krankheiten, die mit einer erhöhten Enzymaktivität dieser Enzyme verbunden sind, dienen.

Die Verbindungen der Formeln I und II können zur Herstellung von Arzneimitteln zur Behandlung von Schädigungen nach Ischämien und zur Prophylaxe bei erwarteten Ischämien verschiedener Organe eingesetzt werden.

Die vorliegenden Benzimidazole der allgemeinen Formel I und II können danach zur Behandlung und Prophylaxe von neurodegenerativen Krankheiten, die nach Ischämie, Trauma (Schädel-Hirntrauma), Massenblutungen, Subarachnoidal-Blutungen und Stroke auftreten, und von neurodegenerativen Krankheiten wie multipler Infarkt-Dementia, Alzheimer Krankheit, Huntington Krankheit und von Epilepsien, insbesondere von generalisierten epileptischen Anfällen, wie zum Beispiel Petit mal und tonisch-clonische Anfälle und partiell epileptischen Anfällen, wie Temporal Lope, und komplex-partiellen Anfällen, und weiterhin zur Behandlung und Prophylaxe von Schädigungen des Herzens nach cardialen Ischämien und Schädigungen der Nieren nach renalen Ischämien, zum Beispiel der akuten Niereninsuffizienz, des akuten Nierenversagens oder von Schädigungen, die während und nach einer Nierentransplantation auftreten, dienen. Weiterhin können die Verbindungen der allgemeinen Formeln I und II zur Behandlung des akuten Myocardinfarkts und Schädigungen, die während und nach dessen medikamentöser Lyse auftreten (zum Beispiel mit TPA, Reteplase, Streptokinase oder mechanisch mit einem Laser oder Rotablator) und von Mikroinfarkten während und nach Herzklappenersatz, Aneurysmenresektionen und Herztransplantationen dienen. Ebenfalls können die vorliegenden Benzimidazole I bzw. II zur Behandlung einer Revascularisation kritisch verengter Koronaraterien, zum Beispiel bei der PCTA und Bypass-Operationen, und kritisch verengter peripherer Arterien, zum Beispiel Beinarterien, dienen. Zudem können die Benzimidazole I bzw. II bei der Chemotherapie von Tumoren und deren Metastasierung nützlich sein und zur Behandlung von Entzündungen und rheumatischen Erkrankungen, wie z.B. rheumatischer Arthritis dienen.

Die erfindungsgemäßen Arzneimittelzubereitungen enthalten neben den üblichen Arneimittel-Hilfsstoffen eine therapeutisch wirksame Menge der Verbindungen I und II.

Für die lokale äußere Anwendung, zum Beispiel in Puder, Salben oder Sprays, können die Wirkstoffe in den üblichen Konzentrationen enthalten sein. In der Regel sind die Wirkstoffe in einer Menge von 0,001 bis 1 Gew.-%, vorzugsweise 0,001 bis 0,1 Gew.-% enthalten. ,

Bei der inneren Anwendung werden die Präperationen in Einzeldosen verabreicht. In einer Einzeldosis werden pro kg Körpergewicht 0,1 bis 100 mg gegeben. Die Zubereitung können täglich in einer oder mehreren Dosierungen je nach Art und Schwere der Erkrankungen verabreicht werden.

Entsprechend der gewünschten Applikationsart enthalten die erfindungsgemäßen Arzneimittelzubereitungen neben dem Wirkstoff die üblichen Trägerstoffe und Verdünnungsmittel. Für die lokale äußere Anwendung können pharmazeutisch-technische Hilfsstoffe, wie Ethanol, Isopropanol, oxethyliertes Ricinusöl, oxethyliertes Hydriertes Ricinusöl, Polyacrylsäure, Polyethylenglykol, Polyethylenglykostearat, ethoxylierte Fettalkohole, Paraffinöl, Vaseline und Wollfett, verwendet werden. Für die innere Anwendung eignen sich zum Beispiel Milchzucker, Propylenglykol, Ethanol, Stärke, Talk und Polyvinylpyrrolidon.

Ferner können Antioxidationsmittel wie Tocopherol und butyliertes Hydroxyanisol sowie butyliertes Hydroxytoluol**,** geschmacksverbessernde Zusatzstoffe, Stabilisierungs-, Emulgier- und Gleitmittel enthalten sein.

Die neben dem Wirkstoff in der Zubereitung enthaltenen Stoffe sowie die bei der Herstellung der pharmazeutischen Zubereitungen verwendeten Stoffe sind toxikologisch unbedenklich und mit dem jeweiligen Wirkstoff verträglich. Die Herstellung der Arzneimittelzubereitungen erfolgt in üblicher Weise, zum Beispiel durch Vermischung des Wirkstoffes mit anderen üblichen Trägerstoffen und Verdünnungsmitteln.

Die Arzneimittelzubereitungen können in verschiedenen Applikationsweisen verabreicht werden, zum Beispiel peroral, parenteral wie intravenös durch Infusion, subkutan, intraperitoneal und topisch. So sind Zubereitungsformen wie Tabletten, Emulsionen, Infusions- und Injektionslösungen, Pasten, Salben, Gele, Cremes, Lotionen, Puder und Sprays möglich.

### Beispiel A: Hemmung des Enzyms Poly(ADP-ribose)polymerase oder PARP (EC 2.4.2.30)

Eine 96well Mikrotiterplatte (Falcon) wird mit Histonen (Type II-AS; SIGMA H7755) beschichtet. Histone werden dazu in Carbonat-Puffer (0,05 M NaHCO₃; pH 9,4) zu einer Konzentration von 50 µg/ml gelöst. Die einzelnen Wells der Mikrotiterplatte werden über Nacht mit je 100 µl dieser Histon Lösung inkubiert. Anschließend wird die Histon Lösung entfernt und die einzelnen Wells mit 200 µl einer l%igen BSA (Bovine Serum Albumine) Lösung in Carbonat-Puffer für 2 Stunden bei Raumtemperatur inkubiert. Anschließend wird dreimal mit Waschpuffer (0,05 % Tween10 in PBS) gewaschen. Für die Enzymreaktion werden je Well 50 µl der Enzymreaktionslösung (5 µl Reaktions-Puffer (1M Tris-HCl pH 8.0, 100 mM MgCl₂, 10 mM DTT, ) 0,5 µl PARP (c=0,22 µg/µl), 4 µl aktivierte DNA (SIGMA D-4522, 1 mg/ml in Wasser), 40,5 µl H₂O) mit 10 µl einer Inhibitorlösung für 10 Minuten vorinkubiert. Die Enzymreaktion wird durch Zugabe von 40 µl einer Substratlösung (4 µl Reaktion-Puffer (s.o.), 8 µl NAD-Lösung (100 µM in H₂O), 28 µl H₂O) gestartet. Reaktionszeit ist 20 Minuten bei Raumtemperatur. Die Reaktion wird durch dreimaliges Waschen mit Waschpuffer (s.o.) gestoppt. Anschließend folgt eine einstündige Inkubation bei Raumtemperatur mit einem spezifischen Anti-Poly-ADP-Ribose Antikörper inkubiert. Als Antikörper wurden ein monoklonaler anti-Poly-(ADP-ribose) Antikörpern "10H" (Kawamaitsu H et al. (1984) Monoclonal antibodies to poly (adenosine diphosphate ribose) recognize different structures. Biochemistry 23, 3771-3777) verwendet. Polyklonale Antikörper können ebenso verwendet werden.

Die Antikörper wurden in einer 1:5000 Verdünnung in AntikörperPuffer (1%BSA in PBS; 0,05 % Tween20) eingesetzt. Nach dreimaligem Waschen mit Waschpuffer folgt eine einstündige Inkubation bei Raumtemperatur mit dem sekundärem Antikörper. Hier wurden für den monoklonalen Antikörper ein anti-Maus-IgG gekoppelt mit Peroxidase (Boehringer Mannheim) und für den Kaninchen Antikörper ein anti-Rabbit-IgG gekoppelt mit Peroxidase (SIGMA A-6154) jeweils in einer 1:10.000 Verdünnung in Antikörperpuffer verwendet. Nach dreimaligem Waschen mit Waschpuffer erfolgt die Farbreaktion unter Verwendung von 100 µl/Well Farbreagenz (SIGMA, TMB-Fertigmix, T8540) für ca. 15 min. bei Raumtemperatur. Die Farbreaktion wird durch Zugabe von 100 µl 2M H₂SO₄ gestoppt. Danach wird sofort gemessen (450 nm gegen 620 nm; ELISA Platten Lesegerät "Easy Reader" EAR340AT, SLT-Labinstruments, Österreich). Der IC₅₀-Wert eines zu messenden Inhibitors liegt bei der Inhibitorkonzentration, wo eine halbmaximale Farbkonzentrationsänderung auftritt.

### Beispiel B: Bestimmung des Wasserlöslichkeit

Eine zu messende Verbindung wird direkt in einem festgelegten Volumen Wasser gelöst und die entstandene Lösung mit einer Natriumacetat-Lösung auf pH-Wert 5 bis 6 eingestellt, so daß die zu prüfende Konzentration des Wirkstoffs erreicht wird. Fall die Meßsubstanz nicht als wasserlösliches Salz vorliegt, wurde diese in möglichst wenig Dimethylsulfoxid gelöst und anschließend mit Wasser verdünnt (Endkonzentration an Dimethylsulfoxid ≤ 1 %), wonach auch hier der pH-Wert noch eingestellt wurde. Der potente PARP-Inhibitor NU 1076 (WO 97/04771) zeigte hier eine Löslichkeit < 0,01 %, wogegen das erfindungsgemäße Beispiel 1 eine Löslichkeit > 0,5 % aufweist.

### Beispiele

### Beispiel 1

### 2-(cis-4-Amino-1-cyclohexyl)-benzimidazol-4-carbonsäureamid x 2 HCl

### a) 2-Amino-3(cis-4-amino-1-cyclohexyl)amido-benzoesäuremethylester

2,4 g (9,9 mMol) cis-4(N(tert.-Butoxycarbonyl)amido-cyclohexancarbonsäure und 2,7 ml (19,7 mMol) Triethylamin wurden in 70 ml wasserfreiem Tetrahydrofuran gelöst und bei -10°C tropfenweise mit einer Lösung aus 0,94 ml (9,9 mMol) Chlorameisensäureethylester in 25 ml wasserfreiem Tetrahydrofuran versetzt. Alles wurde noch für 1 Stunde bei 0°C gerührt. Danach wurden 1,6 g (9,9 mMol) 2,3-Diaminobenzoesäuremethylester zugegeben und alles für 16 Stunden bei Raumtemperatur gerührt. Das Reaktionsgemisch wurde auf Eiswasser gegossen und mit wäßriger Natriumhydrogenkarbonat-Lösung alkalisiert. Diese wäßrige Phase wurde mit Essigester extrahiert. Die organische Phase wurde noch mit wäßriger Natriumhydrogenkarbonat-Lösung und Wasser gewaschen, getrocknet und im Vakuum eingeengt. Man erhielt 2,7 g des Produktes.

### b) 2-(cis-4-Amino-1-cyclohexyl)-benzimidazol-4-carbonsäuremethylester

2,6 g des Produktes 1a wurden in 80 ml Essigsäure für 1 Stunde unter Rückfluß gekocht. Das Reaktionsgemisch wurde im Vakuum eingeengt und der anfallende Niederschlag zwischen Essigester und wäßriger Natriumkarbonat-Lösung verteilt. Die organische Phase wurde mit Wasser gewaschen, getrocknet und im Vakuum eingeengt. Der anfallende Rückstand wurde noch chromatographisch gereinigt (Fließmittel: Essigester/Methanol = 3/1), wobei man 0,7 g des Produktes erhielt.

### c) 2-(cis-4-Amino-1-cyclohexyl)-benzimidazol-4-carbonsäurehydrazid

0,65 g des Produktes aus der Vorschrift 1b wurden mit 0,6 g (11,9 mMol) Hydrazinhydrat in 5 ml Ethanol für 90 Minuten unter Rückfluß gekocht. Danach wurde alles im Vakuum eingeengt, wobei man ein Rohprodukt erhielt, das ohne weitere Reinigung umgesetzt wurde.

### d) 2-(cis-4-Amino-1-cyclohexyl)-benzimidazol-4-carbonsäureamid x 2 HCl

Das Produkt aus 1c wurde in eine Dispersion aus 2 g Raney-Nickel/Wasser und 20 ml Dimethylformamid zugetropft. Alles wurde für 1 Stunde unter Rückfluß gekocht. Nach dem Abkühlen wurde das Raney-Nickel abfiltriert und das Filtrat im Vakuum eingeengt. Der Rückstand wurde mit Wasser behandelt und der anfallende Niederschlag abgesaugt. Der Niederschlag wurde in Isopropanol gelöst und etherischer Chlorwasserstoffsäure zugesetzt. Der anfallende Niederschlag wurde abgesaugt. Man erhielt 0,19 g des Produktes.
¹H-NMR (D₆-DMSO): δ = 1.7 (2H), 1.9 (4H), 2.2-2.4 (4H), 3.4 (2H), 7.6 (1H) und 7.9 (1H) ppm

### Beispiel 2

### 2 (3-Methoxy-cyclohexyl)-benzimidazol-4-carbonsäureamid

### a) 6-Nitro-2-carboxy-benzamid

52,5 g (0,27 Mol) 3-Nitrophthalsäureanhydrid wurden innerhalb von 30 Minuten bei Raumtemperatur portionsweise in 75 ml konzentriertem Ammoniakwasser eingerührt. Anschließend wurde alles auf 0°C gekühlt, wonach ein Niederschlag auskristallisierte, der abgesaugt wurde. Dieser Niederschlag wurde durch leichtes Erwärmen in 125 ml Wasser gelöst und zügig mit 25,6 ml 32 %iger Salzsäure versetzt. Der Ansatz wurde auf 0°C gekühlt und das ausgefallene Kristallisat abgesaugt. Man erhielt 45 g des Produktes.

### b) 2-Amino-3-nitro-benzoesäure

109 g (1,9 Mol) Kaliumhydroxid wurden in 400 ml Wasser gelöst und bei 0°C tropfte man 11 ml (0,22 Mol) Brom hinzu. Anschließend wurde innerhalb von 1 Stunde 45 g (0,21 Mol) des Zwischenproduktes 2a zugegeben. Die Reaktionslösung wurde noch für 1 Stunde bei 60°C gerührt und danach für 16 Stunden bei Raumtemperatur. Durch Zugabe von Salzsäure wurde der pH-Wert der Lösung auf 5 bis 6 eingestellt, wonach das Produkt ausfällt. Man erhielt 30,8 g des Produktes.

### c) 2-Amino-3-nitro-benzoesäureethylester

30,8 g (0,17 Mol) des Zwischenproduktes 2b wurden in 170 ml Ethanol gegeben und nach vorsichtiger Zugabe von 20 ml konzentrierter Schwefelsäure für 24 Stunden unter Rückfluß gekocht. Anschließend wurde der Ansatz vorsichtig auf ein Eis-/Ammoniakwasser-Gemisch gegeben, wobei das Produkt ausfällt. Man erhielt 28,8 g des Produktes.

### d) 2,3-Diamino-benzoesäureethylester

28,8 g (0,14 Mol) des Zwischenproduktes 2c wurden in 200 ml Ethanol nach Zugabe von 1 g Palladium/Kohle (10 %ig) hydriert. Der Ansatz wurde filtriert und das Filtrat im Vakuum eingeengt. Man erhielt 23,5 g des Produktes.

### e) 2,3-Diaminobenzamid x 2 µHCl

23,5 g (0,13 Mol) des Zwischenproduktes 2d wurden in 200 ml n-Butanol nach Zugabe von 50 ml Hydrazinhydrat für 16 Stunden auf 100°C erwärmt. Anschließend wurde alles im Vakuum eingeengt.

50 g Raney-Nickel wurden in 200 ml Dimethylformamid/Wasser (1/1) suspendiert. Der obige Vakuum-Rückstand wurde vorsichtig (Gasentwicklung!) in diese Suspension gegeben und alles für 8 Stunden auf 100°C erwärmt. Anschließend wurde filtriert und das Filtrat im Vakuum eingeengt. Der Rückstand wurde in wenig Methanol gelöst und bis zur beginnenden Trübung mit Ether versetzt. Es fällt ein Niederschlag aus, der abfiltriert wurde. Das Filtrat wurde im Vakuum eingeengt. Dieser Rückstand wurde erneut in Methanol gelöst und vorsichtig mit isopropanolischer Chlorwasserstoff-Lösung versetzt. Da ausgefallene Produkt wurde abgesaugt. Man erhielt 31 g des Produktes.

### f) 2-(3-Methoxy-cyclohexyl)-benzimidazol-4-carbonsäureamid

1,4 g (8,9 Mol) 3-Methoxycyclohexancarbonsäure wurden analog der Vorschrift 3a mit dem Zwischenprodukt 2e umgesetzt und das so erhaltene Material analog der Vorschrift 3b cyclisiert. Man erhielt 0,2 g des Produktes.
¹H-NMR (D₆-DMSO): δ = 1.1-2.3 (8H), 3.2 (1H), 3.3 (3H), 3.6 (1H), 7.2 (1H), 7.6 (1H), 7.65 (1H), 7.7 (1H) und 9.2 (1H) ppm

### Beispiel 3

### 2(4-Methoxy-cyclohexyl)-benzimidazol-4-carbonsäureamid

### a) 2-Amino-3-(4-methoxycyclohexyl)amido-benzamid

1,4 g (8,9 Mol) 4-Methoxycyclohexancarbonsäure wurden in 30 ml Tetrahydrofuran gelöst und bei 0°C nacheinander mit 1,9 g (1,8 mMol) N'-(3-Dimethylaminopropyl)-N-ethyl-carbodiimid x Hydrochlorid, 1,8 g (11,6 mMol) N-Hydroxybenzotriazol und 1,95 g (19.2 mMol) Triethylamin versetzt. Man ließ für 1 Stunde rühren. Anschließend wurden 2,0 g (8,9 mMol) 2,3-Diaminobenzamid x 2 Hydrochlorid und 1,95 g (19,2 mMol) Triethylamin zugegeben. Danach wurde alles für 1 Stunde noch bei 0°C und 16 Stunden bei Raumtemperatur gerührt. Der Ansatz wurde mit viel Wasser verdünnt und mehrmals mit Essigester extrahiert. Die organischen Phasen wurden vereinigt, getrocknet und im Vakuum eingeengt. Man erhielt 1,5 g des Produktes.

### b) 2-(4-Methoxy-cyclohexyl)benzimidazol-4-carbonsäureamid

1,3 g des Zwischenproduktes 3a wurden in 60 ml konzentrierter Essigsäure für 3 Stunden unter Rückfluß gekocht. Danach wurde alles im Vakuum eingeengt und der Rückstand chromatographisch (Fließmittel: Methanol/Methylenchlorid = 1/20) gereinigt. Man erhielt 0,8 g des Produktes.
¹H-NMR (D₆-DMSO): δ = 1.1-2.3 (8H), 2.9 (1H), 3.0 (1H), 3.25 (3H), 7.1 (1H), 7.5 (1H), 7.6 (1H), 7.75 (1H) und 9.2 (1H) ppm

### Beispiel 4

### 2(4(2-N,N-Diethylaminoeth-1-yloxy)cyclohexyl)-benzimidazol-4-carbonsäureamid x 2 HCl

### a) 4-(2-N,N-Diethylaminoeth-1-yloxy)cyclohexancarbonsäureethylester

Zu 2,7 g (64 mMol) Natriumhydrid in Dimethylformamid wurden bei Raumtemperatur eine Lösung von 1 g (58 mMol) 3-Hydroxycyclohexancarbonsäureethylester in Dimethylformamid zugetropft. Man rührte alles für 30 Minuten. Danach tropfte man 7,8 g (58 mMol) N,N,N(2-Chlor-eth-1-yl)-diethylamin, gelöst in Dimethylformamid, zu und rührte alles für 16 Stunden bei Raumtemperatur. Anschließend gab man vorsichtig wenig Wasser zu und engte dann den Ansatz im Vakuum ein. Der Rückstand wurde zwischen Diethylether und Wasser verteilt, das organische Lösungsmittel getrocknet und im Vakuum eingeengt. Das so erhaltene Rohprodukt wurde noch chromatographisch (Fließmittel:Methanol) gereinigt.

### b) 4-(2-N,N-Diethylaminoeth-1-yloxy)cyclohexancarbonsäure

1,4 g (5,2 mMol) des Zwischenproduktes 4a wurden in 15 ml Wasser/Ethanol (2:1) gegeben und mit 0,4 g (10,3 mMol) Natriumhydroxid versetzt. Alles wurde für 2 Stunden bei Raumtemperatur gerührt und anschließend im Vakuum eingeengt. Man erhielt ein Rohprodukt, das direkt weiter umgesetzt wurde.

### c) 2-Amino-3(4(2-N,N-Diethylaminoeth-1-yloxy)cycloheyl)-amidobenzamid

1,2 g (4,9 mMol) des Zwischenproduktes 4b wurden analog der Vorschrift 3a mit 2,3-Diaminobenzamid x 2 HCl umgesetzt. Das Rohprodukt wurde direkt weiter umgesetzt.

### d) 2(4(2-N,N-Diethylamonoeth-1-yloxy)cyclohexyl)-benzimidazol-4-carbonsäureamid x 2 HCl

Das Rohprodukt aus 4c wurde analog der Vorschrift 3b umgesetzt. Das Produkt wurde chromatographisch (Fließmittel: Methanol/Methylenchlorid = 1/20 + 0,1 % NH₃OH) gereinigt. Das Produkt wurde anschließend in das Hydrochlorid überführt.
¹H-NMR (D₆-DMSO): δ = 1.2 (6H), 1.3-2.3 (8H), 2.9-3.5 (9H), 3.7 (1H), 3.8 (2H), 7.5 (1H), 7.8 (1H), 7.9 (1H), 8.0 (1H), 8.5 (1H) und 10.2 (breit) ppm

### Analog der Vorschriften der Beispiele 1 bis 4 wurden hergestellt:

### Beispiel 5

trans-2(4-Aminocyclohexyl)-benzimidazol-4-carbonsäureamid MS: m/e = 256 (M⁺)

### Beispiel 6

trans-2(4-(Aminomethyl)cyclohexyl)-benzimidazol-4-carbonsäureamid MS: m/e = 270 (M⁺)

### Beispiel 7

2-(4-Methylcyclohexyl)-benzimidazol-4-carbonsäureamid MS: m/e = 257 (M⁺)

### Beispiel 8

2-(3-Methylcyclohexyl)-benzimidazol-4-carbonsäureamid MS: m/e = 257 (M⁺)

### Beispiel 9

2-(2-Methylcyclohexyl)-benzimidazol-4-carbonsäureamid MS: m/e = 257 (M⁺)

### Beispiel 10

2-(3-Benzyloxyamido-cyclohexyl)-benzimidazol-4-carbonsäureamid
¹H-NMR (D₆-DMSO): δ = 1.2-2.3 (8H), 3.1 (1H), 3.5 (1H), 5.0 (2H), 7.2-7.5 (6H), 7.7-7.9 (3H) und 9.4 (1H) ppm

### Beispiel 11

2-(3-Amino-cyclohexyl)-benzimidazol-4-carbonsäureamid x HCl
¹H-NMR (D₆-DMSO): δ = 1.3-2.3 (8H), 3.5 (1H), 3.7 (1H), 7.7 (1H) und 7.9 (2H) ppm

Nach den oben beschrieben Methoden können folgende Verbindungen hergestellt werden:
1. 2-(cis-4-Carboxy-1-cyclohexyl)-benzimidazol-4-carbonsäureamid
2. 2-(trans-4-Carboxy-1-cyclohexyl)-benzimidazol-4-carbonsäureamid
3. 2-(4-tert.-Butyl-1-cyclohexyl)-benzimidazol-4-carbonsäureamid
4. 2-(2,4,6-Trimethyl-1-cyclohexyl)-benzimidazol-4-carbonsäureamid
5. 2-(3-Amino-2-methyl-1-cyclohexyl)-benzimidazol-4-carbonsäureamid
6. 2-(2-Hydroxy-1-cyclohexyl)-benzimidazol-4-carbonsäureamid
7. 2-(trans-4-n-Pentan-1-yl-1-cyclohexyl)-benzimidazol-4-carbonsäureamid
8. 2-(4-Hydroxy-1-cyclohexyl)-benzimidazol-4-carbonsäureamid
9. 2-(cis-3-Amino-1-cyclohexyl)-benzimidazol-4-carbonsäureamid
10. 2-(trans-3-Amino-1-cyclohexyl)-benzimidazol-4-carbonsäureamid
11. 2-(4-n-Propan-1-yl-1-cyclohexyl)-benzimidazol-4-carbonsäureamid
12. 2-(4-n-Butan-1-yl-1-cyclohexyl)-benzimidazol-4-carbonsäureamid
13. 2-(4-tert.-Butyl-2-methyl-1-cyclohexyl)-benzimidazol-4-carbonsäureamid
14. 2- (3-Carboxy-1-cyclohexyl)-benzimidazol-4-carbonsäureamid
15. 2-(cis-2-Amino-1-cyclohexyl)-benzimidazol-4-carbonsäureamid
16. 2-(3-Hydroxy-1-cyclohexyl)-benzimidazol-4-carbonsäureamid
17. 2-(4-Trifluormethyl-1-cyclohexyl)-benzimidazol-4-carbonsäureamid
18. 2-(2.6-Dimethyl-4-hydroxy-1-cyclohexyl)-benzimidazol-4-carbonsäureamid
19. 2-(4-Amino-2.6-dimethyl-1-cyclohexyl)-benzimidazol-4-carbonsäureamid
20. 2-(4-Phenyl-1-cyclohexyl)-benzimidazol-4-carbonsäureamid
21. 2-(4-(4-Chlorphenyl)-1-cyclohexyl)-benzimidazol-4-carbonsäureamid
22. 2-(trans-4-(tert.Butoxycarbonylaminomethyl)-1-cyclohexyl)-benzimidazol-4-carbonsäureamid
23. 2-(4-Amidinomethyl-1-cyclohexyl)-benzimidazol-4-carbonsäureamid
24. 2-(cis-4-Carboxy-1-cyclohexyl)-benzimidazol-4-carbonsäureamid
25. 2-(4-(Diethylamino)-1-cyclohexyl)-benzimidazol-4-carbonsäureamid
26. 2-(4-(Dimethylamino)-1-cyclohexyl)-benzimidazol-4-carbonsäureamid
27. 2-(3-(Diethylamino)-1-cyclohexyl)-benzimidazol-4-carbonsäureamid
28. 2-(3-(Dimethylamino)-1-cyclohexyl)-benzimidazol-4-carbonsäureamid
29. 2-(4-(Dimethylamino)-1-cyclohexyl)-benzimidazol-4-carbonsäureamid
30. 2-(cis-4-(Diethylamino)-1-cyclohexyl)-benzimidazol-4-carbonsäureamid
31. 2-(trans-4-(Diethylamino)-1-cyclohexyl)-benzimidazol-4-carbonsäureamid
32. 2-(cis-4-(Dimethylamino)-1-cyclohexyl)-benzimidazol-4-carbonsäureamid
33. 2-(trans-4-(Dimethylamino)-1-cyclohexyl)-benzimidazol-4-carbonsäureamid
34. 2-(4-(Ethylamino)-1-cyclohexyl)-benzimidazol-4-carbonsäureamid
35. 2-(cis-4-(Ethylamino)-1-cyclohexyl)-benzimidazol-4-carbonsäureamid
36. 2-(trans-4-(Ethylamino)-1-cyclohexyl)-benzimidazol-4-carbonsäureamid
37. 2-(4-(Methylamino)-1-cyclohexyl)-benzimidazol-4-carbonsäureamid
38. 2-(cis-4-(Methylamino)-1-cyclohexyl)-benzimidazol-4-carbonsäureamid
39. 2-(trans-4-(Methylamino)-1-cyclohexyl)-benzimidazol-4-carbonsäureamid
40. 2-(4-(Propylamino)-1-cyclohexyl)-benzimidazol-4-carbonsäureamid
41. 2-(cis-4-(Propylamino)-1-cyclohexyl)-benzimidazol-4-carbonsäureamid
42. 2-(trans-4-(Propylamino)-1-cyclohexyl)-benzimidazol-4-carbonsäureamid
43. 2-(3-(Ethylamino)-1-cyclohexyl)-benzimidazol-4-carbonsäureamid
44. 2-(3-(Methylamino)-1-cyclohexyl)-benzimidazol-4-carbonsäureamid
45. 2-(3-(Propylamino)-1-cyclohexyl)-benzimidazol-4-carbonsäureamid
46. 2-(4-(N,N-Ethylmethylamino)-1-cyclohexyl)-benzimidazol-4-carbonsäureamid
47. 2-(3-(N,N-Ethylmethylamino)-1-cyclohexyl)-benzimidazol-4-carbonsäureamid
48. 2-(4-(N,N-Ethylpropylamino)-1-cyclohexyl)-benzimidazol-4-carbonsäureamid
49. 2-(3-(N,N-Ethylpropylamino)-1-cyclohexyl)-benzimidazol-4-carbonsäureamid
50. 2-(4-Piperidin-1-yl-1-cyclohexyl)-benzimidazol-4-carbonsäureamid
51. 2-(3-Piperidin-1-yl-1-cyclohexyl)-benzimidazol-4-carbonsäureamid
52. 2-(cis-4-Piperidin-1-yl-1-cyclohexyl)-benzimidazol-4-carbonsäureamid
53. 2-(trans-4-Piperidin-1-yl-1-cyclohexyl)-benzimidazol-4-carbonsäureamid
54. 2-(4-Pyrrolidin-1-yl-1-cyclohexyl)-benzimidazol-4-carbonsäureamid
55. 2-(3-Pyrrolidin-1-yl-1-cyclohexyl)-benzimidazol-4-carbonsäureamid
56. 2-(cis-4-Pyrrolidin-1-yl-1-cyclohexyl)-benzimidazol-4-carbonsäureamid
57. 2-(trans-4-Pyrrolidin-1-yl-1-cyclohexyl)-benzimidazol-4-carbonsäureamid
58. 2-(4-(4-Methylpiperazin-1-yl-1-cyclohexyl)-benzimidazol-4-carbonsäureamid
59. 2-(3-(4-Methylpiperazin-1-yl-1-cyclohexyl)-benzimidazol-4-carbonsäureamid
60. 2-(cis-4-(4-Methylpiperazin-1-yl-1-cyclohexyl)-benzimidazol-4-carbonsäureamid
61. 2-(trans-4-(4-Methylpiperazin-1-yl-1-cyclohexyl)-benzimidazol-4-carbonsäureamid
62. 2-(4-(Piperazin-1-yl-1-cyclohexyl)-benzimidazol-4-carbonsäureamid
63. 2-(3-(Piperazin-1-yl-1-cyclohexyl)-benzimidazol-4-carbonsäureamid
64. 2-(cis-4-(Piperazin-1-yl-1-cyclohexyl)-benzimidazol-4-carbonsäureamid
65. 2-(trans-4-(Piperazin-1-yl-1-cyclohexyl)-benzimidazol-4-carbonsäureamid
66. 2-(4-(4-Benzyl-piperazin-1-yl-1-cyclohexyl)-benzimidazol-4-carbonsäureamid
67. 2-(3-(4-Benzylpiperazin-1-yl-1-cyclohexyl)-benzimidazol-4-carbonsäureamid
68. 2-(4-(4-Phenyl-piperazin-1-yl-1-cyclohexyl)-benzimidazol-4-carbonsäureamid
69. 2-(3-(4-Phenylpiperazin-1-yl-1-cyclohexyl)-benzimidazol-4-carbonsäureamid
70. 2-(4-(4-Propylpiperazin-1-yl-1-cyclohexyl)-benzimidazol-4-carbonsäureamid
71. 2-(3-(4-Propylpiperazin-1-yl-1-cyclohexyl)-benzimidazol-4-carbonsäureamid
72. 2-(4-(4-Butylpiperazin-1-yl-1-cyclohexyl)-benzimidazol-4-carbonsäureamid
73. 2-(3-(4-Butylpiperazin-1-yl-1-cyclohexyl)-benzimidazol-4-carbonsäureamid
74. 2-(4-(4-Homopiperazin-1-yl-1-cyclohexyl)-benzimidazol-4-carbonsäureamid
75. 2-(3-(4-Homopiperazin-1-yl-1-cyclohexyl)-benzimidazol-4-carbonsäureamid
76. 2-(4-(4-(N-Methylhomopiperazin-1-yl)-1-cyclohexyl)-benzimidazol-4-carbonsäureamid
77. 2-(3-(4-(N-Methylhomopiperazin-1-yl)-1-cyclohexyl)-benzimidazol-4-carbonsäureamid
78. 2-(4-(4-(4-Phenyl-1,2,5,6-tetrahydropyridin-1-yl-1-cyclohexyl)-benzimidazol-4-carbonsäureamid
79. 2-(3-(4-(4-Phenyl-1,2,5,6-tetrahydropyridin-1-yl-1-cyclohexyl)-benzimidazol-4-carbonsäureamid

## Patentansprüche

1. Verbindungen der Formel I oder II 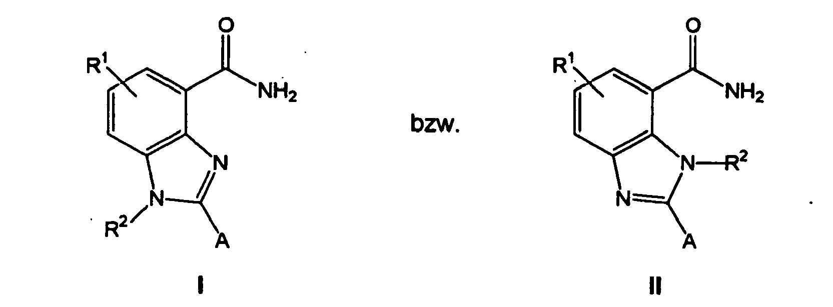 worin
A einen gesättigten oder einfach ungesättigten Carbozyklus mit 3 bis 8 Kohlenstoffatomen bedeutet, der zusätzlich noch einen Benzolring ankondensiert haben kann, wobei die Ringe noch mit ein oder zwei unterschiedlichen oder gleichen Resten R³ sowie dem Rest R⁴ substituiert sein können, und
R¹ Wasserstoff, und
R² Wasserstoff, verzweigtes und unverzweigtes C₁-C₆-Alkyl, C₁-C₄-Alkyl-Phenyl, und
R³ C₁-C₆-Alkyl, OH, O-C₁-C₄-Alkyl, O-C₁-C₄-Alkyl-Phenyl, NR¹¹R¹², Phenyl, C₁-C₄-Alkyl-Phenyl, CF₃, COOH, COOC₁-C₄-Alkyl, CONH-C₁-C₄-Alkyl, CONH₂, wobei die Phenyl-Ringe noch mit maximal zwei gleichen oder unterschiedlichen Resten R³¹ substituiert sein können, wobei
R¹¹ und R¹² unabhängig voneinander Wasserstoff oder C₁-C₄-Alkyl bedeuten und
R³¹ OH, C₁-C₆-Alkyl, O-C₁-C₄-Alkyl, Chlor, Brom, Jod, Fluor, CF₃, Nitro, NR¹¹R¹², und
R⁴ -(O)ₚ-(CH₂)_{q}-B bedeutet, wobei
B NR⁴¹R⁴² und 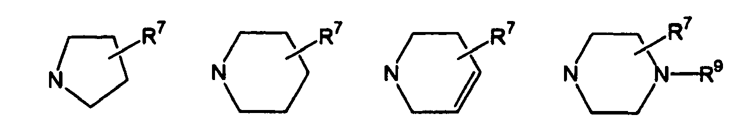 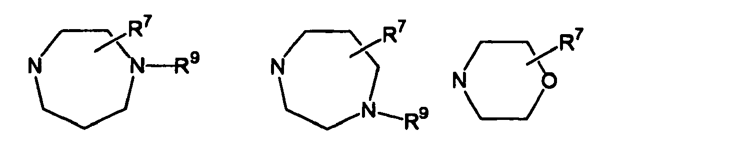 bedeutet, wobei
p 0 und 1 bedeuten kann und
q 0, 1, 2 oder 3 sein kann, wobei wenn q = 0 ist auch p = 0 ist, und
R⁴¹ Wasserstoff, C₁-C₆-Alkyl, (CH₂)ᵣ-E und
R⁴² Wasserstoff, C₁-C₆-Alkyl, -CO-R⁸, SO₂-R⁸, -(C=N)-R⁸ und -(C=N)-NHR⁸ und
r 0, 1, 2, 3, 4 und
E Phenyl, der noch maximal zwei Reste R⁷² tragen kann, und, wenn r ≠ 0, 1 ist, auch NR¹¹R¹², NH-C₁-C₄-Alkyl-Phenyl, Pyrrolidin, Piperidin, Dihydropiperidin, Morpholin, Homopiperidin, Piperazin, das noch mit C₁-C₆-Alkyl und C₁-C₄-Alkyl-Phenyl substituiert sein kann, und Homopiperazin, das noch mit C₁-C₆-Alkyl und C₁-C₄-Alkyl-Phenyl substituiert sein kann, und
R⁷ Wasserstoff, C₁-C₆-Alkyl, Phenyl, wobei der Ring noch mit bis zu zwei gleichen oder unterschiedlichen Resten R⁷¹ substituiert sein kann, und
R⁷¹ OH, C₁-C₆-Alkyl, O-C₁-C₄-Alkyl, Chlor, Brom, Jod, Fluor, CF₃, Nitro, NR¹¹R¹², NHOC₁-C₄-Alkyl und
R⁷² OH, C₁-C₆-Alkyl, O-C₁-C₄-Alkyl, Chlor, Brom, Jod, Fluor, CF₃, Nitro, NR¹¹R¹², NHOC₁-C₄-Alkyl und
R⁸ C₁-C₆-Alkyl, Phenyl, C₁-C₄-Alkyl-Phenyl-O-C₁-C₄-Alkyl-Phenyl, wobei die Ringe noch mit bis zu zwei gleichen oder unterschiedlichen Resten R⁸¹ substituiert sein können, und
R⁸¹ OH, C₁-C₆-Alkyl, O-C₁-C₄-Alkyl, Chlor, Brom, Jod, Fluor, CF₃, Nitro, NR¹¹R¹², und
R⁹ Wasserstoff, C₁-C₆-Alkyl, C₁-C₄-Alkyl-Phenyl, Phenyl, wobei die Ringe noch mit bis zu zwei Resten R⁹¹ substituiert sein können, und
R⁹¹ OH, C₁-C₆-Alkyl, O-C₁-C₄-Alkyl, Chlor, Brom, Jod, Fluor, CF₃, Nitro, NR¹¹R¹² sein kann,
sowie ihre tautomeren Formen, möglichen enantiomeren und diastereomeren Formen, möglichen cis-trans-Isomeren an den Ringen in A und deren Phosphate, Carbamate von Aminosäuren und Ester.

2. Verbindungen nach Anspruch 1, wobei A mindestens mit einem Substituenten R³ oder R⁴ substituiert ist.

3. Verbindungen der Formel I oder II nach einem der Ansprüche 1 oder 2 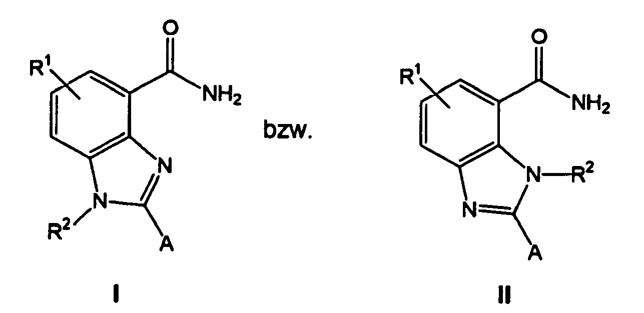 worin
A Tetralin, Indan, Cycloheptan, Cyclopentan, Cyclobutan und Cyclopropan bedeutet.

4. Verbindungen nach Anspruch 1, wobei
A Cyclohexan, und
R¹, R² und R³ Wasserstoff, und
R⁴ die Bedeutung wie im Anspruch 1 hat, wobei p 0 und 1 und q 0, 1 und 2,
R⁴¹ und R⁴², unabhängig voneinander, Wasserstoff und C₁-C₄-Alkyl bedeutet,
R⁷ Wasserstoff, C₁-C₄-Alkyl und Phenyl,
R⁹ Wasserstoff, C₁-C₄-Alkyl und C₁-C₂-Alkyl-Phenyl, und
R⁴ in 3- und 4-Stellung am Cyclohexan-Ring stehen kann, wobei sowohl die cis- und trans-Formen als auch deren Gemische eingeschlossen sind.

5. Arzneimittel enthaltend neben üblichen Trägem und Hilfsstoffen Verbindungen der Formel I oder 11 nach einem der Ansprüche 1 bis 4.

6. Verbindungen der Formel I bzw. II nach einem der Ansprüche 1 bis 4 zur Verwendung in der Behandlung von neurodegenerativen Krankheiten und neuronalen Schädigungen.

7. Verbindungen nach Anspruch 6 zur Verwendung in der Behandlung von solchen neurodegenerativen Krankheiten und neuronalen Schädigungen, die durch Ischämie, Trauma oder Massenblutungen ausgelöst werden.

8. Verbindungen nach Anspruch 6 zur Verwendung in der Behandlung des Schlaganfalls und des Schädel-Himtraumas.

9. Verbindungen nach Anspruch 6 zur Verwendung in der Behandlung der Alzheimerschen Krankheit, der Parkinsonsche Krankheit und der Huntington-Krankheit.

10. Verbindungen der Formel I bzw. II nach einem der Ansprüche 1 bis 4 zur Verwendung in der Behandlung oder Prophylaxe von Schädigungen durch Ischämien.

11. Verbindungen der Formel I bzw. II nach einem der Ansprüche 1 bis 4 zur Verwendung in der Behandlung von Epilepsien.

12. Verbindungen nach Anspruch 11 zur Verwendung in der Behandlung von generalisierten epileptischen Anfällen, partiell epileptischen Anfällen und komplex-partiellen Anfällen.

13. Verbindungen nach Anspruch 12 zur Verwendung in der Behandlung von Petit mal und tonisch-clonische Anfällen sowie Temporal Lope.

14. Verbindungen der Formel I bzw. II nach einem der Ansprüche 1 bis 4 zur Verwendung in der Behandlung von Schädigungen der Nieren nach renalen Ischämien und zur Behandlung während und nach Nierentransplantationen.

15. Verbindungen der Formel I bzw. II nach einem der Ansprüche 1 bis 4 zur Verwendung in der Behandlung von Schädigungen des Herzens nach cardialen Ischämien.

16. Verbindungen der Formel I bzw. II nach einem der Ansprüche 1 bis 4 zur Verwendung in der Behandlung von Mikroinfarkten.

17. Verbindungen nach Anspruch 16, wobei die Mikroinfarkte während und nach Herzklappenersatz, Aneurysmenresektionen und Herztransplantationen auftreten.

18. Verbindungen der Formel I bzw. II nach einem der Ansprüche 1 bis 4 zur Verwendung in der Behandlung bei einer Revasculariation kritischer verengter Koronararterien.

19. Verbindungen nach Anspruch 18 bei PTCA und Bypass-Operationen oder kritisch verengter peripherer Arterien.

20. Verbindungen nach Anspruch 19, worin die kritisch verengte periphere Arterien Beinarterien sind.

21. Verbindungen der Formel I bzw. II nach einem der Ansprüche 1 bis 4 zur Verwendung in der Behandlung des akuten Myocardinfarktes und von Schädigungen während und nach dessen medikamentöser oder mechanischer Lyse.

22. Verbindungen der Formel I bzw. II nach einem der Ansprüche 1 bis 4 zur Verwendung in der Behandlung von Tumoren und deren Metastasierung.

23. Verbindungen der Formel I bzw. II nach einem der Ansprüche 1 bis 4 zur Verwendung in der Behandlung von Sepsis und des septischen Schocks.

24. Verbindungen der Formel I bzw. II nach einem der Ansprüche 1 bis 4 zur Verwendung in der Behandlung von immunologischen Krankheiten und rheumatischen Erkrankungen.

25. Verbindungen nach Anspruch 24 zur Verwendung in der Behandlung von Entzündungen und rheumatoider Arthritis.

26. Verbindungen der Formel I bzw. II nach einem der Ansprüche 1 bis 4 zur Verwendung in der Behandlung von Diabetes mellitus.

27. Verwendung von Verbindungen der Formel I bzw. II nach einem der Ansprüche 1 bis 4 zur Herstellung von Arzneimitteln zur Behandlung von neurodegenerativen Krankheiten und neuronalen Schädigungen, Schädigungen durch Ischämien, Epilepsien, Schädigungen der Nieren nach renalen Ischämien und zur Behandlung während und nach Nierentransplantationen, Schädigungen des Herzens nach cardialen Ischämien, Mikroinfarkten, einer Revasculariation kritischer verengter Koronararterien, akuten Myocardinfarktes und Schädigungen während und nach dessen medikamentöser oder mechanischer Lyse, Tumoren und deren Metastasierung, Sepsis, septischen Schock, immunologischen Krankheiten, rheumatischen Erkrankungen und Diabetes mellitus.

## Claims

1. Compounds of formula I or II 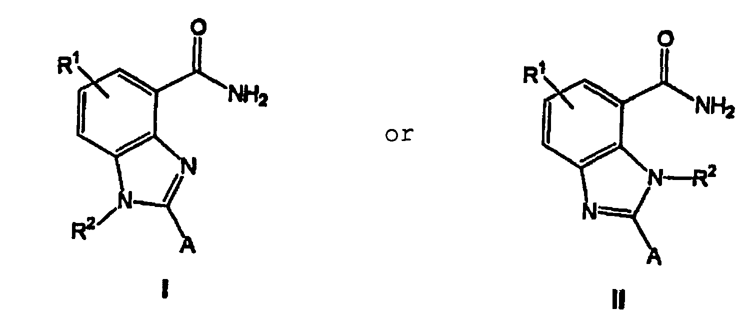 in which
A denotes a saturated or monounsaturated carbocyclic system which has from 3 to 8 carbon atoms and can additionally have a fused-on benzene ring, it being possible for the rings also to be substituted by one or two different or identical radicals R³ and also the radical R⁴, and
R¹ denotes hydrogen, and
R² denotes hydrogen, branched and unbranched C₁-C₆-alkyl, C₁-C₄-alkyl-phenyl, and
R³ denotes C₁-C₆-alkyl, OH, O-C₁-C₄-alkyl, O-C₁-C₄-alkyl-phenyl, NR¹¹R¹², phenyl, C₁-C₄-alkyl-phenyl, CF₃, COOH, COOC₁-C₄-alkyl, CONH-C₁-C₄-alkyl, CONH₂, it being possible for the phenyl rings also to be substituted by not more than two identical or different radicals R³¹, wherein R¹¹ and R¹² independently of one another denote hydrogen or C₁-C₄-alkyl, and
R³¹ denotes OH, C₁-C₆-alkyl, O-C₁-C₄-alkyl, chlorine, bromine, iodine, fluorine, CF₃, nitro, NR¹¹R¹², and
R⁴ denotes -(O)ₚ-(CH₂)_{q}-B, wherein
B denotes NR⁴¹R⁴² and 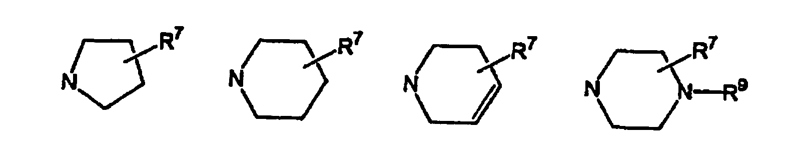 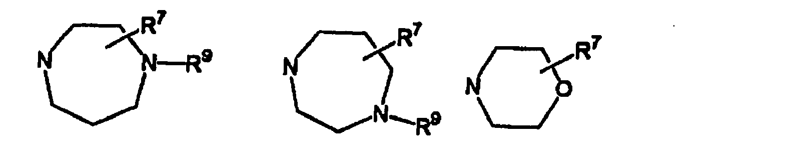 wherein
p can denote 0 and 1 and
q can be 0, 1, 2 or 3, wherein when q is 0 p is also 0, and
R⁴¹ denotes hydrogen, C₁-C₆-alkyl, (CH₂)ᵣ-E and
R⁴² denotes hydrogen, C₁-C₆-alkyl, -CO-R⁸, SO₂-R⁸, -(C=N)-R⁸ and - (C=N) -NHR⁸ and
r denotes 0, 1, 2, 3, 4 and
E denotes phenyl, which can also carry not more than two radicals R⁷², and, when r # 0, 1, also NR¹¹R¹², NH-C₁-C₄-alkyl-phenyl, pyrrolidine, piperidine, dihydropiperidine, morpholine, homopiperidine, piperazine, which can also be substituted by C₁-C₆-alkyl and C₁-C₄-alkyl-phenyl, and homopiperazine, which can also be substituted by C₁-C₆-alkyl and C₁-C₄-alkyl-phenyl, and
R⁷ denotes hydrogen, C₁-C₆-alkyl, phenyl, it being possible for the ring also to be substituted by up to two identical or different radicals R⁷¹, and
R⁷¹ denotes OH, C₁-C₆-alkyl, O-C₁-C₄-alkyl, chlorine, bromine, iodine, fluorine, CF₃, nitro, NR¹¹R*¹²* NHOC₁-C₄-alkyl, and
R⁷² denotes OH, C₁-C₆-alkyl, O-C₁-C₄-alkyl, chlorine, bromine, iodine, fluorine, CF₃, nitro, NR¹¹R¹², NHOC₁-C₄-alkyl, and
R⁸ denotes C₁-C₆-alkyl, phenyl, C₁-C₄-alkyl-phenyl-O-C₁-C₄-alkyl-phenyl, it being possible for the rings also to be substituted by up to two identical or different radicals R⁸¹, and
R⁸¹ denotes OH, C₁-C₆-alkyl, O-C₁-C₄-alkyl, chlorine, bromine, iodine, fluorine, CF₃, nitro, NR¹¹R¹², and
R⁹ denotes hydrogen, C₁-C₆-alkyl, C₁-C₄-alkyl-phenyl, phenyl, it being possible for the rings also to be substituted by up to two radicals R⁹¹, and
R⁹¹ can be OH, C₁-C₆-alkyl, O-C₁-C₄-alkyl, chlorine, bromine, iodine, fluorine, CF₃, nitro, NR¹¹R¹²,
and their tautomeric forms, possible enantiomeric and diastereoisomeric forms, possible cis-trans isomers on the rings in A, and their phosphates, carbamates of amino acids and esters.

2. Compounds according to claim 1, wherein A is substituted at least by a substituent R³ or R⁴.

3. Compounds of formula I or II according to either claim 1 or claim 2 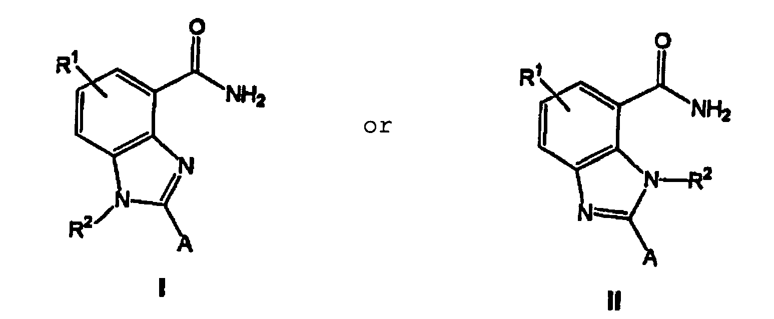 wherein
A denotes tetralin, indane, cycloheptane, cyclopentane, cyclobutane and cyclopropane.

4. Compounds according to claim 1, wherein
A denotes cyclohexane, and
R¹, R² and R³ denote hydrogen, and
R⁴ has the meaning given in claim 1, wherein p denotes 0 and 1 and q denotes 0, 1 and 2,
R⁴¹ and R⁴², independently of one another, denote hydrogen and C₁-C₄-alkyl,
R⁷ denotes hydrogen, C₁-C₄-alkyl and phenyl,
R⁹ denotes hydrogen, C₁-C₄-alkyl and C₁-C₂-alkylphenyl, and
R⁴ can be in the 3- and 4-position on the cyclohexane ring, both the cis and trans forms and mixtures thereof being included.

5. Medicament comprising compounds of formula I or II according to any one of claims 1 to 4 in addition to conventional carriers and auxiliary substances.

6. Compounds of formula I or II according to any one of claims 1 to 4 for use in the treatment of neurodegenerative diseases and neuronal damage.

7. Compounds according to claim 6 for use in the treatment of neurodegenerative diseases and neuronal damage induced by ischaemia, trauma or massive bleeding.

8. Compounds according to claim 6 for use in the treatment of stroke and craniocerebral trauma.

9. Compounds according to claim 6 for use in the treatment of Alzheimer's disease, Parkinson's disease and Huntington's disease.

10. Compounds of formula I or II according to any one of claims 1 to 4 for use in the treatment or prophylaxis of damage caused by ischaemia.

11. Compounds of formula I or II according to any one of claims 1 to 4 for use in the treatment of epilepsy.

12. Compounds according to claim 11 for use in the treatment of generalised epileptic seizures, partial epileptic seizures and complex partial seizures.

13. Compounds according to claim 12 for use in the treatment of petit mal and tonoclonic seizures as well as temporal lobe.

14. Compounds of formula I or II according to any one of claims 1 to 4 for use in the treatment of kidney damage following renal ischaemia and for treatment during and after kidney transplants.

15. Compounds of formula I or II according to any one of claims 1 to 4 for use in the treatment of heart damage following cardiac ischaemia.

16. Compounds of formula I or II according to any one of claims 1 to 4 for use in the treatment of microinfarcts.

17. Compounds according to claim 16, wherein the microinfarcts occur during and after heart valve replacement, aneurysm resections and heart transplants.

18. Compounds of formula I or II according to any one of claims 1 to 4 for use in treatment in the case of revascularisation of critically narrowed coronary arteries.

19. Compounds according to claim 18 in PTCA and bypass operations or critically narrowed peripheral arteries.

20. Compounds according to claim 19, wherein the critically narrowed peripheral arteries are leg arteries.

21. Compounds of formula I or II according to any one of claims 1 to 4 for use in the treatment of acute myocardial infarct and of damage during and after the medical or mechanical lysis thereof.

22. Compounds of formula I or II according to any one of claims 1 to 4 for use in the treatment of tumours and metastases thereof.

23. Compounds of formula I or II according to any one of claims 1 to 4 for use in the treatment of sepsis and septic shock.

24. Compounds of formula I or II according to any one of claims 1 to 4 for use in the treatment of immunological diseases and rheumatic diseases.

25. Compounds according to claim 24 for use in the treatment of inflammations and rheumatoid arthritis.

26. Compounds of formula I or II according to any one of claims 1 to 4 for use in the treatment of diabetes mellitus.

27. Use of compounds of formula I or II according to any one of claims 1 to 4 in the preparation of medicaments for the treatment of neurodegenerative diseases and neuronal damage, damage caused by ischaemia, epilepsy, kidney damage following renal ischaemia and for treatment during and after kidney transplants, heart damage following cardiac ischaemia, microinfarcts, revascularisation of critically narrowed coronary arteries, acute myocardial infarct and damage during and after the medical or mechanical lysis thereof, tumours and metastases thereof, sepsis, septic shock, immunological diseases, rheumatic diseases and diabetes mellitus.

## Revendications

1. Composés de la formule I ou II 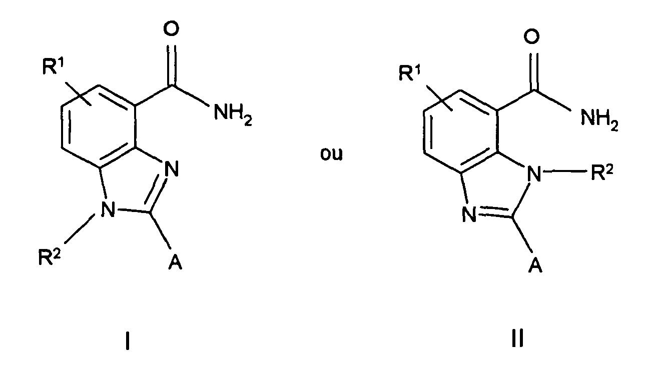 dans lesquelles
A représente un carbocycle saturé ou une fois insaturé avec de 3 à 8 atomes de carbone, lequel peut encore présenter un noyau benzène condensé, les noyaux pouvant encore être substitués avec un ou deux radicaux R³ identiques ou différents ainsi qu'avec le radical R⁴, et
R¹ représente un atome d'hydrogène, et
R² représente un atome d'hydrogène, un groupe alkyle en C₁-C₆ linéaire et ramifié, un (alkyle en C₁-C₄)-phényle, et
R³ représente un groupe alkyle en C₁-C₆, OH, O-(alkyle en C₁-C₄), O-(alkyle en C₁-C₄)-phényle, NR¹¹R¹², phényle, (alkyle en C₁-C₄)-phényle, CF₃, COOH, COO-(alkyle en C₁-C₄), CONH-(alkyle en C₁-C₄), CONH₂, les noyaux phényle pouvant encore être substitués avec au maximum deux radicaux R³¹ identiques ou différents,
R¹¹ et R¹² représentant indépendamment l'un de l'autre un atome d'hydrogène ou un groupe alkyle en C₁-C₄ et
R³¹ représente OH, un groupe alkyle en C₁-C₆, O-(alkyle en C₁-C₄), un atome de chlore, de brome, d'iode, de fluor, CF₃, un groupe nitro, NR¹¹R¹², et
R⁴ représente -(O)ₚ-(CH₂)_{q}-B, où
B représente NR⁴¹R⁴² et 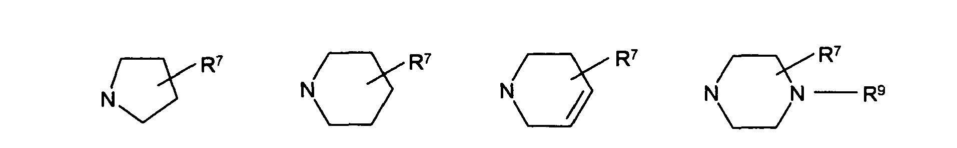 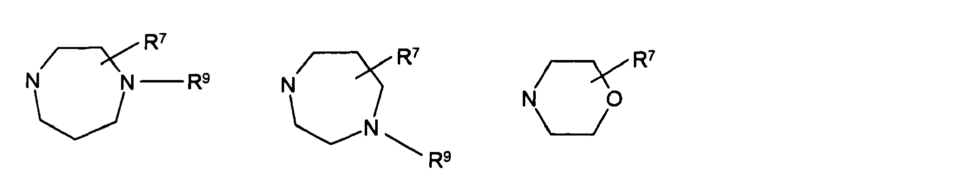 où
p peut représenter 0 et 1 et
q peut être égal à 0,1, 2 ou 3, lorsque q = 0 p est également = 0, et
R⁴¹ représente un atome d'hydrogène, un groupe alkyle en C₁-C₆, (CH₂)ᵣ-E et
R⁴² représente un atome d'hydrogène, un groupe alkyle en C₁-C₆, -CO-R⁸, SO₂-R⁸, -(C=N)-R⁸ et -(C=N)-NHR⁸ et
r est égal à 0, 1, 2, 3, 4 et
E représente un groupe phényle qui peut encore porter au maximum deux radicaux R⁷², et, lorsque r ≠ 0, 1, également NR¹¹R¹², un groupe NH-(alkyle en C₁-C₄)-phényle, pyrrolidine, pipéridine, dihydropipéridine, morpholine, homopipéridine, pipérazine, lequel peut encore être substitué avec un groupe alkyle en C₁-C₆ et (alkyle en C₁-C₄)-phényle, et homopipérazine, lequel peut encore être substitué avec un groupe alkyle en C₁-C₆ et (alkyle en C₁-C₄)-phényle, et
R⁷ représente un atome d'hydrogène, un groupe alkyle en C₁-C₆, phényle, le noyau pouvant encore être substitué avec jusqu'à deux radicaux R⁷¹ identiques ou différents, et
R⁷¹ représente OH, un groupe alkyle en C₁-C₆, O-(alkyle en C₁-C₄), un atome de chlore, de brome, d'iode, de fluor, CF₃, un groupe nitro, NR¹¹R¹², NHO(alkyle en C₁-C₄) et
R⁷² représente OH, un groupe alkyle en C₁-C₆, O-(alkyle en C₁-C₄), un atome de chlore, de brome, d'iode, de fluor, CF₃, un groupe nitro, NR¹¹R¹², NHO(alkyle en C₁-C₄) et
R⁸ représente un groupe alkyle en C₁-C₆, phényle, (alkyle en C₁-C₄)-phényle-O-(alkyle en C₁-C₄)-phényle, les noyaux pouvant encore être substitués avec jusqu'à deux radicaux R⁸¹ identiques ou différents, et
R⁸¹ représente OH, un groupe alkyle en C₁-C₆, O-(alkyle en C₁-C₄), un atome de chlore, de brome, d'iode, de fluor, CF₃, un groupe nitro, NR¹¹R¹², et
R⁹ représente un atome d'hydrogène, un groupe alkyle en C₁-C₆, (alkyle en C₁-C₄)-phényle, phényle, les noyaux pouvant encore être substitués avec jusqu'à deux radicaux R⁹¹, et
R⁹¹ peut être OH, un groupe alkyle en C₁-C₆, O-(alkyle en C₁-C₄), un atome de chlore, de brome, d'iode, de fluor, CF₃, un groupe nitro, NR¹¹R¹²,
ainsi que leurs formes tautomères, formes énantiomères et diastéréomères possibles, isomères cis-trans possibles sur les noyaux dans A et leurs phosphates, carbamates d'aminoacides et esters.

2. Composés selon la revendication 1, dans lesquels A est au moins substitué avec un substituant R³ ou R⁴.

3. Composés de la formule I ou II selon l'une quelconque des revendications 1 ou 2 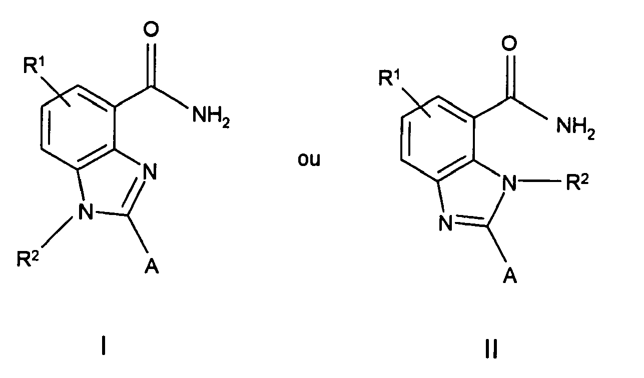 dans lesquelles
A représente la tétraline, l'indane, le cycloheptane, le cyclopentane, le cyclobutane et le cyclopropane.

4. Composés selon la revendication 1, dans lesquels
A représente le cyclohexane, et
R¹, R² et R³ représentent un atome d'hydrogène, et
R⁴ a la signification comme dans la revendication 1, p étant égal à 0 et à 1 et q étant égal à 0, 1 et 2,
R⁴¹ et R⁴², indépendamment l'un de l'autre, représentent un atome d'hydrogène et un groupe alkyle en C₁-C₄,
R⁷ représente un atome d'hydrogène, un groupe alkyle en C₁-C₄ et phényle,
R⁹ représente un atome d'hydrogène, un groupe alkyle en C₁-C₄ et (alkyle en C₁-C₂)-phényle, et
R⁴ peut être en position 3 et 4 sur le noyau cyclohexane, les formes cis et trans ainsi qu'également leurs mélanges étant inclus.

5. Médicament contenant en plus des supports et auxiliaires classiques des composés de la formule I et II selon l'une quelconque des revendications 1 à 4.

6. Composés de la formule I respectivement II selon l'une quelconque des revendications 1 à 4 pour une utilisation dans le traitement de maladies neurodégénératives et de lésions neuronales.

7. Composés selon la revendication 6 pour une utilisation dans le traitement de maladies neurodégénératives et de lésions neuronales qui sont occasionnées par des ischémies, des traumatismes ou des hémorragies.

8. Composés selon la revendication 6 pour une utilisation dans le traitement de attaque cérébrale et du traumatisme crânien.

9. Composés selon la revendication 6 pour une utilisation dans le traitement de la maladie d'Alzheimer, de la maladie de Parkinson et de la maladie de Huntington.

10. Composés de la formule I respectivement II selon l'une quelconque des revendications 1 à 4 pour une utilisation dans le traitement ou la prophylaxie de lésions dues à des ischémies.

11. Composés de la formule I respectivement II selon l'une quelconque des revendications 1 à 4 pour une utilisation dans le traitement des épilepsies.

12. Composés selon la revendication 11 pour une utilisation dans le traitement de crises épileptiques généralisées, de crises épileptiques partielles et de crises complexes-partielles.

13. Composés selon la revendication 12 pour une utilisation dans le traitement de petit mal et de crises toniques-cloniques ainsi que de Temporal Lope.

14. Composés de la formule 1 respectivement II selon l'une quelconque des revendications 1 à 4 pour une utilisation dans le traitement de lésions rénales après des ischémies rénales et pour le traitement pendant et après des transplantations rénales.

15. Composés de la formule I respectivement II selon l'une quelconque des revendications 1 à 4 pour une utilisation dans le traitement de lésions cardiaques après des ischémies cardiaques.

16. Composés de la formule I respectivement II selon l'une quelconque des revendications 1 à 4 pour une utilisation dans le traitement de microinfarctus.

17. Composés selon la revendication 16, dans lesquels les microinfarctus interviennent pendant et après le remplacement de valvulves cardiaques, des résections d'anévrismes et des transplantations cardiaques.

18. Composés de la formule I respectivement II selon l'une quelconque des revendications 1 à 4 pour une utilisation dans le traitement lors d'une revascularisation d'artères coronaires rétrécies de manière critique.

19. Composés selon la revendication 18 pour des opérations PTCA et de pontage ou des artères périphériques rétrécies de manière critique.

20. Composés selon la revendication 19, dans lesquels les artères périphériques rétrécies de manière critique sont des artères de la jambe.

21. Composés de la formule I respectivement II selon l'une quelconque des revendications 1 à 4 pour une utilisation dans le traitement de l'infarctus du myocarde aigu et de lésions pendant et après leurs lyses médicamenteuses ou mécaniques.

22. Composés de la formule I respectivement II selon l'une quelconque des revendications 1 à 4 pour une utilisation dans le traitement de tumeurs et de leurs métastases.

23. Composés de la formule I respectivement II selon l'une quelconque des revendications 1 à 4 pour une utilisation dans le traitement de la septicémie et du choc septique.

24. Composés de la formule I respectivement II selon l'une quelconque des revendications 1 à 4 pour une utilisation dans le traitement de maladies immunologiques et de maladies rhumatismales.

25. Composés selon la revendication 24 pour une utilisation dans le traitement d'inflammations et d'arthrites rhumatoïdes.

26. Composés de la formule I respectivement II selon l'une quelconque des revendications 1 à 4 pour une utilisation dans le traitement du diabète sucré.

27. Utilisation de composés de la formule I respectivement II selon l'une quelconque des revendications 1 à 4 pour la préparation de médicaments destinés au traitement de maladies neurodégénératives et de lésions neuronales, de lésions dues aux ischémies, d'épilepsies, de lésions rénales après des ischémies rénales et pour le traitement pendant et après des transplantations rénales, de lésions cardiaques après des ischémies cardiaques, de microinfarctus, d'une revascularisation d'artères coronaires rétrécies de manière critique, d'infarctus du myocarde aigus et de lésions pendant et après leurs lyses médicamenteuses ou mécaniques, de tumeurs et de leurs métastases, de la septicémie, de chocs septiques, de maladies immunologies, de maladies rhumatismales et du diabète sucré.
